# EUROPEAN PATENT SPECIFICATION

(11) **EP 4 343 316 B1**
(45) Date of publication and mention of the grant of the patent: **22.04.2026**
(21) Application number: 23821472.0
(22) Date of filing: 16.06.2023
(51) Int. Cl.: G01N 27/26, G01N 27/327, C12Q 1/00

(54) **USE OF NATURAL POLYMER COMPOUND IN BIOSENSER**
VERWENDUNG EINER NATÜRLICHEN POLYMERVERBINDUNG IN EINEM BIOSENSOR
UTILISATION D'UN COMPOSÉ POLYMÈRE NATUREL DANS UN BIODÉTECTEUR

(30) Priority: 20.06.2022 CN 202210698681
(43) Date of publication of application: 27.03.2024
(73) Proprietor: Shanghai United Imaging Microelectronics Technology Co., Ltd., Shanghai 201899 (CN)
(72) Inventor: LIU, Liangling, Shanghai 201899 (CN); LIN, Jing, Shanghai 201899 (CN)
(74) Representative: Wang, Bo
(86) International application number: PCT/CN2023/100831
(87) International publication number: WO 2023/246661

(56) References cited:
- EP-B1- 0 214 805
- WO-A2-01/27626
- CN-A- 102 363 776
- CN-A- 104 833 713
- CN-A- 108 872 215
- CN-A- 111 796 016
- CN-A- 114 027 834
- CN-A- 115 165 982
- DE-C1- 19 545 547
- JP-A- H09 152 414
- KR-A- 20200 062 944
- US-A1- 2019 290 170
- CULICA M E ET AL: "Cellulose-based biogenic supports, remarkably friendly biomaterials for proteins and biomolecules", BIOSENSORS AND BIOELECTRONICS, vol. 182, 113170, 19 March 2021 (2021-03-19), XP086544376, DOI: 10.1016/J.BIOS.2021.113170
- SHUNGUANG WANG, JI XINSONG, YUAN ZHONGYI: "Study of Cellulose Acetate Membrane-based Glucose Biosensor", CHINESE JOURNAL OF BIOTECHNOLOGY, vol. 11, no. 3, 27 March 1995 (1995-03-27), pages 260 - 265, XP093120686
- "Master's Theses ", 7 April 2020, WUHAN INSTITUTE OF TECHNOLOGY, China, article XIA, JIAN: "Functional Design of Cellulose Membranes and Its Application in Biosensor Field ", pages: 1 - 113, XP009551431, DOI: 10.27727/d.cnki.gwhxc.2020.000078

## Description

### CROSS-REFERENCE TO RELATED APPLICATIONS

The present disclosure claims priority to Chinese patent application No. 202210698681.8, filed on June 20, 2022.

### TECHNICAL FIELD

The present disclosure relates to the technical field, and in particular, to applications of natural polymer compounds in biosensors.

### BACKGROUND

A biosensor is used for the determination of substances in living organisms, and its use has become increasingly widespread. For example, the biosensor can be used for in vivo blood glucose determination. However, many implantable biosensors are susceptible to immune reactions in vivo and tend to have a short lifetime. In addition, an existing process for immobilizing a sensitive moiety in the biosensor applies some cross-linking agents with biotoxicity, such as glutaraldehyde, photoinitiator, or acrylic, which not only affects the activity and stability of the sensitive moiety, but also leaves residual cross-linking agents which may enhance a rejection reaction in the human body, thereby reducing a service life of biosensor, and is not conducive to safety of batch production. Relevant prior art is disclosed in CN111796016A, WO01/27626A2, and EP0214805 B1. CN111796016A discloses a method for detecting glucose using a paper-based electrochemical/colorimetric dual-mode biosensor comprising a bioanode and a biocathode. WO01/27626A2 relates to a system and corresponding method for carrying out an amperometric immunoassay of a target antigen in which a solid phase support is provided, having an oxidised cellulose paper on which is immobilised an antibody that specifically binds to target antigen and at least one enzyme. EP0214805 B1 relates to a sensor including a reactive monomolecular film formed on the surface of the gate electrode, and a sensing material fixed on the gate electrode through the reactive monomolecular film by the chemical bond.

### SUMMARY

The invention is set out in the appended set of claims.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating a flexible electrode in a biosensor according to some embodiments of the present disclosure;
FIG. 2 is a schematic diagram illustrating a cross-section of a working electrode in a flexible electrode according to some embodiments of the present disclosure;
FIG. 3A is a schematic diagram illustrating sensitivity test results of a biosensor according to some embodiments of the present disclosure;
FIG. 3B is a diagram illustrating stability test results of a biosensor according to some embodiments of the present disclosure;
FIG. 4A is a diagram illustrating sensitivity test results of another biosensor according to some embodiments of the present disclosure;
FIG. 4B is a diagram illustrating stability test results of another biosensor according to some embodiments of the present disclosure;
FIG. 5A is a diagram illustrating sensitivity test results of another biosensor according to some embodiments of the present disclosure;
FIG. 5B is a diagram illustrating stability test results of another biosensor according to some embodiments of the present disclosure;
FIG. 6 is a diagram illustrating cytotoxicity test results of a working electrode according to some embodiments of the present disclosure.

Reference signs: 100-flexible electrode; 10-flexible base; 20-working electrode; 201-metal layer; 202-enzyme membrane layer; 203-protective layer; 30-reference electrode; 40-counter electrode.

### DETAILED DESCRIPTION

In order to illustrate the technical solutions related to the embodiments of the present disclosure, a brief introduction of the drawings referred to in the description of the embodiments is provided below. Obviously, the drawings described below are only some examples or embodiments of the present disclosure. Those skilled in the art, without further creative efforts, may apply the present disclosure to other similar scenarios according to these drawings. Unless apparent from the locale or otherwise stated, like reference numerals represent similar structures or operations throughout the several views of the drawings.

It should be understood that the term "system," "device," "unit," and/or "module" used herein are one method to distinguish different components, elements, parts, sections, or assemblies of different levels in ascending order. However, the terms can be displaced by another expression if they achieve the same purpose.

As used in the disclosure and the appended claims, the singular forms "a," "an," and/or "the" may include plural forms unless the content clearly indicates otherwise. In general, the terms "comprise," "comprises," and/or "comprising," "include," "includes," and/or "including," merely prompt to include steps and elements that have been clearly identified, and these steps and elements do not constitute an exclusive listing. The methods or devices may also include other steps or elements.

Unless otherwise defined, all technical and scientific terms used herein have the same meaning as commonly understood by those skilled in the art belonging to the present disclosure. The terms used herein in the specification of the present disclosure are for the purpose of describing specific embodiments only and are not intended to limit the invention. The term "and/or" as used herein includes any and all combinations of one or more of the relevant listed items.

A biosensor is a device capable of converting one or more biological signals into one or more readable electrical signals for detecting and monitoring biological processes such as chemicals, bioactive molecules, and metabolic activities of cells in living organisms, and is widely used in the fields of healthcare, environmental monitoring, and food safety. In some embodiments, the biosensor of the present disclosure may include a biometric module, a signal conversion module, an energy supply module, a data processing module, and an output module.

The biometric module usually includes sensitive moieties such as antibodies, enzymes, nucleic acids, cells, etc. During the working process of the biosensor, a specific recognition occurs between the sensitive moiety in the biometric module and the molecule to be tested and some physical or chemical event that leads to a change in one or more chemical signals is generated. The molecule to be tested may include a biomolecule, a metabolite, a drug, or a toxin. For example, the biomolecule may include proteins, nucleic acids, enzymes, cytokines, etc. The metabolite may include glucose, uric acid, lactic acid, ethanol, etc.

A signal converter in the signal conversion module may convert the one or more chemical signals into the one or more readable electrical signals. The signal converter includes an electrochemical converter, an optical converter, a piezoelectric converter, and a thermal converter. The working principle of an electrochemical converter includes generating a current or potential difference through an electrochemical reaction between the biometric module and an electrode. The current or potential difference may be measured and recorded by a detection system.

The energy supply module refers to a module that provides energy for a bio-sensing process. Energy supply manners of the energy supply module include the use of purely chemical energy (e.g., oxygen), solar energy, motion energy, or wireless electromagnetic radiation.

The data processing module may further process the electrical signal in the signal conversion module into signals that are more easily understood by a user. The data processing module may perform operations including electrical signal collection, electrical signal processing, etc.

The output module may present collection results processed by the data processing module to the user. The user includes but is not limited to, personnel of medical and healthcare organizations, personal health managers, athletes, sports enthusiasts, personnel of environmental monitoring organizations, and production or quality supervisors of food or beverages.

One of the embodiments of the present disclosure provides a biosensor. The biosensor includes a working electrode, and the working electrode includes an enzyme membrane layer. The enzyme membrane layer includes a sensitive moiety immobilized with an oxidized natural polymer compound as a cross-linking agent, and the oxidized natural polymer compound is obtained by oxidizing a natural polymer compound.

In some embodiments, the biosensor further includes at least one of a flexible base, a reference electrode, and a counter electrode. In some embodiments, a thickness of the flexible base is within a range of 10 µm to 300 µm. In some embodiments, a material of the flexible base includes at least one of polyimide and polyethylene terephthalate.

In some embodiments, the working electrode further includes at least one of a metal layer and a protective layer.

In some embodiments, the metal layer of the working electrode includes a first metal layer, a second metal layer, and a third metal layer in order from inside to outside. The first metal layer is at least one of Cr, Ti, and Ni-Cr. The second metal layer is at least one of gold, copper, and silver; and the third metal layer is at least one of platinum, platinum-iridium, and platinum-carbon.

In some embodiments, the protective layer includes at least one of polyurethane, polyethylene glycol, cellulose acetate, polyvinyl alcohol, polyvinylpyrrolidone, and polyethylene.

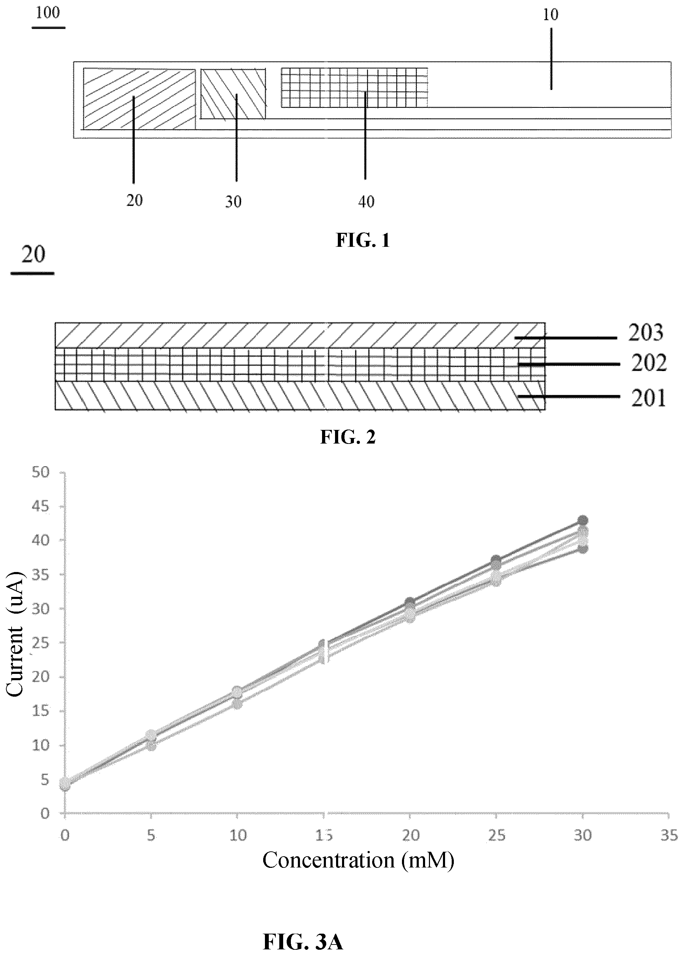

For example, as shown in FIGS. 1 and 2, the biosensor includes a flexible electrode 100. In some embodiments, the flexible electrode 100 includes a flexible base 10 and a working electrode 20, a reference electrode 30, and a counter electrode 40 disposed on the same surface of the flexible base 10.

In some embodiments, a metal layer may be covered on the flexible base 10 through a micro-nanofabrication technique and the metal layer is patterned to form a three-electrode system of the working electrode 20, the reference electrode 30, and the counter electrode 40.

The micro-nanofabrication technique refers to a series of technologies based on semiconductor processes, including photolithography, developing, magnetron sputtering, wet etching, plasma cleaning, laser cutting, etc. The machining precision may be improved by preparing the flexible electrode through the micro-nanofabrication technique.

Referring to FIG. 2, in some embodiments, the working electrode 20 includes a metal layer 201, an enzyme membrane layer 202, and a protective layer 203 disposed in layers. The metal layer 201 is disposed between the flexible base 10 and the protective layer 203.

In some embodiments, the metal layer 201 includes a first metal portion (not shown in the figure), a second metal portion (not shown in the figure), and a third metal portion (not shown in the figure) disposed in layers. In some embodiments, materials of the first metal portion include chromium, titanium, or a nickel-chromium alloy, materials of the second metal portion include gold, copper, or silver, and materials of the third metal portion include platinum, a platinum-iridium alloy, or a platinum-carbon mixture. The second metal portion has a good electrical conductivity to improve the electron transmission efficiency of the biosensor, and the third metal portion has a catalytic effect on the hydrogen peroxide to reduce the effect of hydrogen peroxide that is generated during enzyme catalysis on a catalytic reaction.

A thickness of the enzyme membrane layer is within a range of 1 µm to 5 µm. In some embodiments, a spin-coating process may be utilized to control the thickness of the enzyme membrane layer by controlling the rotational speed of a spin-coating machine. In some embodiments, an effective control of the thickness of the enzyme membrane layer may be implemented by adjusting the amount of cross-linking agent and a cross-linking temperature.

The "natural polymer compound" of the present disclosure refers to a compound that may be produced by living organisms themselves through biochemical action or photosynthesis. Compared with other synthetic organic compounds, the natural polymer compound is more biocompatible because the natural polymer compound originates from living organisms, which greatly reduces the occurrence of immune rejection after entering the human body. The natural polymer compound contains multiple repeating structural units. For example, sodium alginate is a natural polymer compound consisting of β-D-mannuronic acid (M) and α-L-glucuronic acid (G) residues linked by a 1,4-glycosidic bond, which are repetitively arranged to form MM fragments, GG fragments, or MG fragments.

In some embodiments, the natural polymer compound includes a natural polysaccharide polymer compound.

The "polysaccharide" herein refers to a natural polymer compound formed by the dehydration and condensation of a plurality of monosaccharide molecules and bonded through glycosidic bonds. In some embodiments, the polysaccharide polymer compounds include plant polysaccharides (e.g., cellulose, hemicellulose, starch, pectin), animal polysaccharides (e.g., chitin, chitosan, heparin, chondroitin sulfate), agar polysaccharides (e.g., agar, alginic acid, carrageenan), bacterial polysaccharides (e.g., D-glucan, D-galactosan, mannans), and microbial polysaccharides (e.g., dextran, xanthan gum, curdled lactose, pullulan).

In some embodiments, the natural polymer compound contains a hydroxyl group.

In some embodiments, the natural polymer compound includes at least one of cellulose and its derivatives, sodium alginate, chitosan and its derivatives, hyaluronic acid, konjac mannan, starch and its derivatives, pectin, and carrageenan.

In some embodiments, the mechanical strength of the natural polymer compound is relatively high, and after the natural polymer compound is served as the the cross-linking agent to immobilize the sensitive moiety, the stability of the working electrode is relatively great. In some embodiments, the natural polymer compound includes the sodium alginate or the konjac mannan.

In some embodiments, an oxidized natural polymer compound may be obtained by oxidizing the natural polymer compound through an oxidizing agent. The oxidizing agent includes but is not limited to, metal oxides and salts thereof (e.g., chromium reagent, potassium permanganate, manganese dioxide), nitric acid, periodate, periodate, or dimethyl sulfoxide.

In some embodiments, the hydroxyl group in the oxidized natural polymer compound is at least partially oxidized and forms an aldehyde group.

The term "at least partially oxidized" is used herein and refers to that at least one hydroxyl group of the natural polymer compound is oxidized. "Partially" may refer to a proportion of the hydroxyl groups. For example, "partially " may be one-half, one-fifth, or one-tenth, respectively representing that a count of the oxidized hydroxyl groups is one-half, one-fifth, or one-tenth of the total number of hydroxyl groups of the natural polymer compound. In some embodiments, an oxidation degree of the natural polymer compound may be controlled by controlling the concentration or types of the oxidizing agent.

In some embodiments, the oxidized natural polymer compound includes a dialdehyde natural polymer compound.

As used herein, "dialdehyde" refers to that two hydroxyl groups on a structural unit of the natural polymer compound are oxidized to aldehyde groups. For example, by performing a selective oxidation on the hydroxyl groups of the cellulose glucose unit C2-C3 by periodate, the hydroxyl groups on C2-C3 are oxidized to the aldehyde groups, and dialdehyde cellulose is obtained. In some embodiments, at least one of the sodium alginate, the chitosan and its derivatives, the hyaluronic acid, the konjac mannan, the starch and its derivatives, the pectin, and the carrageenan may be mixed with the oxidizing agent to obtain at least one of dialdehyde sodium alginate, dialdehyde chitosan and its derivatives, dialdehyde hyaluronic acid, dialdehyde konjac mannan, dialdehyde starch and its derivatives, dialdehyde pectin, and dialdehyde carrageenan.

In some embodiments, the oxidation degree of the oxidized natural polymer compound is within a range of 5% to 85%.

The oxidation degree refers to the proportion of hydroxyl groups in the oxidized natural polymer compound that are oxidized to aldehyde groups. The oxidation degree of the oxidized natural polymer compound may be expressed by the content of the aldehyde group. For example, if the content of the aldehyde group is 6%, the oxidation degree of the oxidized natural polymer compound is 6%. The content of the aldehyde group of the oxidized natural polymer compound may be determined by using, for example, a chemical titration method, and the method for determining the oxidation degree is not specifically limited herein.

In some embodiments, the oxidation degree of the oxidized natural polymer compound is within a range of 5% to 60%. In some embodiments, the oxidation degree of the oxidized natural polymer compound is within a range of 20% to 70%. In some embodiments, the oxidation degree of the oxidized natural polymer compound is 30% to 80%. In some embodiments, the oxidation degree of the oxidized natural polymer compound is within a range of 5%, 20%, 30%, 40%, 50%, 60%, or 85%. In some embodiments, the oxidation degree of the oxidized natural polymer compound is within a range of 30% to 60%. In some embodiments, the oxidation degree of the oxidized natural polymer compound is 35%, 45%, or 55%.

The sensitive moiety refers to an enzyme.

In some embodiments, the sensitive moiety may be a compound with a primary amino group. In some embodiments, the sensitive moiety includes one of the glucose oxidase, the lactate oxidase, the uricase, or the alcohol oxidase. In some embodiments, the sensitive moiety is the glucose oxidase.

In some embodiments, the enzyme membrane layer further includes an enzyme stabilizer. The enzyme stabilizer is a substance used to enhance the stability and activity of an enzyme, protect the enzyme from environmental factors (e.g., temperature, pH, oxygen, etc.), and prolong service life and the activity of the enzyme. The enzyme stabilizer includes, but is not limited to, polyethylene glycol (PEG), proteins, peptides, amino acids, cholesterol, etc.

The thickness of the enzyme membrane layer is within a range of 1 µm to 5 µm. In some embodiments, the thickness of the enzyme membrane layer may be 1 µm, 2 µm, 3 µm, 4 µm, 5 µm.

In some embodiments, an enzyme membrane containing the glucose oxidase is used as a receptor of the biometric module, and an oxygen electrode is used as the energy supply module to constitute a glucose oxidase biosensor. When the biosensor is placed into a solution to be tested, dissolved oxygen within the solution to be tested and glucose to be tested penetrate the enzyme membrane at the same time, and in the presence of oxygen, the glucose to be tested is catalytically oxidized by the glucose oxidase to form gluconic acid, and at the same time, the dissolved oxygen is consumed to generate hydrogen peroxide, at this time, the concentration of oxygen in the solution declined, the signal conversion module converts this process into one or more electrical signals, the one or more electrical signals are processed by the data processing module, and then the output module outputs a concentration value of the glucose in the solution to be tested.

The embodiments of the present disclosure obtain the oxidized natural polymer compound by oxidizing the natural polymer compound, and use the oxidized natural polymer compound as the cross-linking agent to immobilize the sensitive moiety (e.g., glucose oxidase) in the working electrode of the biosensor to ensure the activity and the stability of the sensitive moiety, which avoids the biotoxicity of a conventional chemical cross-linking agent, and improves the biocompatibility of the working electrode.

One of the embodiments of the present disclosure provides a preparation method for a biosensor, the method includes: obtaining the oxidized natural polymer compound by performing an oxidation operation on the natural polymer compound and immobilizing the sensitive moiety in the working electrode of the biosensor using the oxidized natural polymer compound as the cross-linking agent.

In some embodiments, obtaining the oxidized natural polymer compound by performing the oxidation operation on the natural polymer compound includes:

S11, the natural polymer compound is dissolved in a first solvent such that a natural polymer compound solution is obtained.

In some embodiments, the natural polymer compound includes at least one of the cellulose and its derivatives, the sodium alginate, the chitosan and its derivatives, the hyaluronic acid, the konjac mannan, the starch and its derivatives, the pectin, and the carrageenan. The first solvent refers to a solvent capable of dissolving the natural polymer compound. In some embodiments, the first solvent may include an organic solvent (e.g., ethanol), an acidic solvent, or an alkaline solvent. In some embodiments, the first solvent includes water.

S12, an oxidizing solution is obtained by dissolving the oxidizing agent in a second solvent. The second solvent refers to a solvent capable of dissolving the oxidizing agent. In some embodiments, the second solvent includes water.

In some embodiments, the oxidizing agent includes sodium periodate. In some embodiments, the oxidizing solution is obtained by dissolving the sodium periodate in water.

S13, the oxidized natural polymer compound is obtained based on an oxidation reaction performed by mixing the natural polymer compound solution with the oxidizing solution.

In some embodiments, the oxidized natural polymer compound may be the dialdehyde natural polymer compound. In some embodiments, a sugar ring in the natural polymer compound is oxidized by the oxidizing agent (e.g., the sodium periodate) and an oxidized polymer compound including two aldehyde groups is obtained.

In some embodiments, the oxidation reaction is terminated by adding a terminating agent. In some embodiments, the terminating agent includes ethylene glycol.

In some embodiments, oxidized natural polymer compounds of different oxidation degrees are obtained by controlling the proportion or mixing time between the oxidizing agent and the natural polymer compound.

In some embodiments, the oxidized natural polymer compound is obtained based on the oxidation reaction between the natural polymer compound solution and the oxidizing solution performed for 0.5h-48h. In some embodiments, the oxidized natural polymer compound is obtained based on the oxidation reaction between the natural polymer compound solution and the oxidizing solution performed for 8h-24h. In some embodiments, the oxidized natural polymer compound is obtained based on the oxidation reaction between the natural polymer compound solution and the oxidizing solution performed for 1h, 4h, 10h, 12h, 15h, or 24h.

In some embodiments, an initial reaction concentration of the polymer compound in a mixed solution is within a range of 0.1% to 20% after mixing the natural polymer compound solution with the oxidizing solution. In some embodiments, the initial reactive concentration of the polymer compound in the mixed solution is 0.1%, 0.5%, 5%, 10%, or 20%. In some embodiments, the initial reactive concentration of the polymer compound in the mixed solution is within a range of 1% to 5%. In some embodiments, the initial reaction concentration of the polymer compound in the mixed solution is 1% or 3%.

In some embodiments, the initial reaction concentration of the oxidizing agent in the mixed solution is within a range of 0.1% to 40% after mixing the natural polymer compound solution with the oxidizing solution. In some embodiments, the initial reactive concentration of the oxidizing agent in the mixed solution is 0.1%, 5%, 10%, 20%, or 40%. In some embodiments, the initial reaction concentration of the oxidizing agent in the mixed solution is within a range of 1% to 10%. In some embodiments, the initial reaction concentration of the oxidizing agent in the mixed solution is 0.3%, 0.5%, 1%, 3%, 8%, or 10%.

In some embodiments, the oxidation degree of the oxidized natural polymer compound is within a range of 5% to 85%.

In some embodiments, the oxidation degree of the oxidized natural polymer compound is within a range of 5% to 60%. In some embodiments, the oxidation degree of the oxidized natural polymer compound is within a range of 20% to 70%. In some embodiments, the oxidation degree of the oxidized natural polymer compound is 30% to 80%. In some embodiments, the oxidation degree of the oxidized natural polymer compound is within a range of 5%, 20%, 30%, 40%, 50%, 60%, or 85%. In some embodiments, the oxidation degree of the oxidized natural polymer compound is 30% to 60%. In some embodiments, the oxidation degree of the oxidized natural polymer compound is 35%, 45%, or 55%.

The embodiments of the present disclosure control the oxidation degree of the final oxidized natural polymer compound by adjusting the initial reaction concentration of the oxidizing agent, the initial reaction concentration of the natural polymer compound in the mixed solution, and the time of the oxidation reaction. In such cases, the count of the sensitive moieties is controlled to ensure that the biosensor is in the appropriate sensing sensitivity interval.

In some embodiments, the preparation method of the biosensor includes: immobilizing the sensitive moiety in a working electrode using the oxidized natural polymer compound as a cross-linking agent.

In some embodiments, the working electrode includes the enzyme membrane layer, and the enzyme membrane layer includes the sensitive moiety immobilized using the oxidized natural polymer compound.

In some embodiments, the oxidized natural polymer compound is obtained by oxidizing the natural polymer compound. In some embodiments, the natural polymer compound includes at least one of the cellulose and its derivatives, the sodium alginate, the chitosan and its derivatives, the hyaluronic acid, the konjac mannan, the starch and its derivatives, the pectin, and the carrageenan.

In some embodiments, the sensitive moiety may be a compound with a primary amino group. In some embodiments, the sensitive moiety includes one of the glucose oxidase, the lactate oxidase, the uricase, or the alcohol oxidase.

In some embodiments, a preparation method of the biosensor further includes a method for preparing the enzyme membrane layer. In some embodiments, the method of preparing the enzyme membrane layer includes: obtaining a mixed solution by mixing the enzyme stabilizer, the sensitive moiety, and the oxidized natural polymer compound in the third solvent, and obtaining the enzyme membrane layer based on a cross-linking reaction performed by coating the mixed solution on a metal layer. In some embodiments, the third solvent includes water.

In some embodiments, the sensitive moiety includes the glucose oxidase.

In some embodiments, the enzyme stabilizer includes serum proteins (e.g., bovine serum proteins), serum enzymes, or other proteins with an enzyme stabilizing function.

In some embodiments, the cross-linking reaction includes a Schiff-base reaction between a primary amino group(s) in the glucose oxidase and the aldehyde group(s) in the oxidized natural polymer compound.

In some embodiments, in the mixed solution, a concentration of the sensitive moiety in the mixed solution is within a range of 0.5% to 20%, a concentration of the oxidized natural polymer compound is within a range of 0.1% to 20%, and a concentration of the enzyme stabilizer is within a range of 0.5% to 20%. In some embodiments, in the mixed solution, the concentration of the sensitive moiety is within a range of 5% to 20%, the concentration of the oxidized natural polymer compound is within a range of 1% to 20%, and a concentration of the enzyme stabilizer is within a range of 5% to 20%. In some embodiments, in the mixed solution, the concentration of the sensitive moiety is within a range of 5% to 10%, the concentration of the oxidized natural polymer compound is within a range of 10% to 20%, and the concentration of the enzyme stabilizer is within a range of 5% to 10%. In some embodiments, in the mixed solution, the concentration of the sensitive moiety is within a range of 2% to 10%, the concentration of the oxidized natural polymer compound is within a range of 5% to 15%, and the concentration of the enzyme stabilizer is within a range of 0.5% to 5%.

In some embodiments, the temperature of the cross-linking reaction is within a range of 0°C to 37°C. In some embodiments, the temperature of the cross-linking reaction is within a range of 5°C to 15°C. In some embodiments, the temperature of the cross-linking reaction is within a range of 0°C to 10°C. In some embodiments, the temperature of the cross-linking reaction is 4°C.

The preparation of the enzyme membrane layer is illustrated as an example with the sensitive moiety being the glucose oxidase and the enzyme stabilizer being the serum protein. The preparation method of the enzyme membrane layer 202 includes:

S21, the serum protein, the glucose oxidase, and the oxidized natural polymer compound are mixed in a solvent to obtain a glucose oxidase mixed solution. In some embodiments, the solvent is water.

In some embodiments, the serum protein and the glucose oxidase in different proportions are formulated into an aqueous solution of a certain concentration, and then the oxidized natural polymer compound is formulated into an aqueous solution of a certain concentration, and the glucose oxidase mixed solution is obtained by mixing the two aqueous solutions.

In some embodiments, in the glucose oxidase mixed solution, a concentration of serum protein is within a range of 0.5% to 20%, the concentration of glucose oxidase is within a range of 0.5% to 20%, and the concentration of oxidized natural polymer compound is within a range of 0.1% to 20%.

In S22, an enzyme membrane layer 202 is obtained based on the cross-linking reaction performed by coating the mixed solution of glucose oxidase on the metal layer.

In some embodiments, the glucose oxidase mixed solution obtained by mixing is immediately coated on the metal layer for cross-linking at a certain temperature for a certain period, so that the primary amino group in the glucose oxidase undergoes a Schiff-base reaction with the aldehyde group in the oxidized natural polymer compound to form a stabilized enzyme membrane layer 202. It should be understood that the cross-linking time required to cross-link at different temperatures is different.

In some embodiments, the temperature at which the glucose oxidase mixed solution undergoes the cross-linking reaction on the metal layer is within a range of 0°C to 37°C. In some embodiments, the temperature at which the glucose oxidase mixed solution undergoes the cross-linking reaction on the metal layer is 4°C.

Because the biocompatibility of the natural polymer compound is relatively good, the natural polymer compound is often used in the field of materials, specifically, for the preparations of hydrogels, human tissue scaffolds, etc. However, when the natural polymer compound is applied in the biosensor, the thickness of the enzyme membrane layer may be increased due to the properties of the polymer, and considering a need to control the thickness of the enzyme membrane layer, parameters such as the dosage and temperature of the cross-linking agent in some embodiments of the present disclosure are effectively controlled, thereby implementing an effective control of the thickness of the enzyme membrane layer. In such cases, the enzyme membrane layer is neither too thick and easy to fall off to affect the stability, nor too thin to affect the sensing performance.

In some embodiments, the materials of the protective layer 203 include at least one of polyurethane, polyethylene glycol, cellulose acetate, polyvinyl alcohol, polyvinylpyrrolidone, and polyethylene.

The protective layer 203 may be prepared in a dip-coating manner. In some embodiments, a protective layer solution is obtained by dissolving a material of the protective layer(i.e., at least one of polyurethane, polyethylene glycol, cellulose acetate, polyvinyl alcohol, polyvinyl pyrrolidone, and polyethylene) in a solvent, and the protective layer is obtained by placing the enzyme membrane layer 202 in the protective layer solution, taking out the enzyme membrane layer 202, and drying the enzyme membrane layer 202.

In some embodiments, the solvent of the protective layer includes at least one of tetrahydrofuran, N, N-dimethylformamide (DMF), cyclohexane, and acetone.

In some embodiments, in the protective layer solution, a concentration of the material of the protective layer (i.e., at least one of polyurethane, polyethylene glycol, cellulose acetate, polyvinyl alcohol, polyvinylpyrrolidone, and polyethylene) is within a range of 0.5% to 10%.

One of the embodiments of the present disclosure also provides a use of a biosensor in bio-sensing. In some embodiments, the biosensor may include at least one of an immune sensor, an enzyme sensor, a nucleic acid sensor, and a cellular sensor.

In some embodiments, the biosensor is used to detect at least one of the glucose, the uric acid, the lactic acid, or the alcoholic substance.

In some embodiments, the biosensor is a glucose biosensor and the sensitive moiety includes the glucose oxidase.

The following embodiments are some more specific illustrations of embodiments related to some of the above embodiments. Some of these embodiments may also be replaced or combined with corresponding elements in other embodiments to form new embodiments. The experimental methods in the following embodiments are conventional if not otherwise specified. The test materials used in the following embodiments are, unless otherwise illustrated, purchased from a conventional biochemical reagent company. The quantitative tests in the following embodiments were repeated three times, and the results were averaged. It should be understood that the following embodiments are intended to better explain the present disclosure and are not intended to limit the invention.

### Embodiment 1

In the first step, an aqueous solution of sodium alginate was obtained by dissolving 3 g of sodium alginate in 150 ml of distilled water.

In the second step, an aqueous solution of sodium periodate was obtained by dissolving 2 g of sodium periodate in 50 ml of distilled water.

In the third step, the aqueous solution of sodium periodate was added dropwise to the aqueous solution of sodium alginate, the reaction was carried out in the dark at room temperature for 24h, then the reaction was terminated by adding 5 ml of ethylene glycol and stirring for 1h, and then the oxidized sodium alginate was obtained by purifying the aqueous solution via dialysis and then drying in an oven.

In the fourth step, an aqueous solution of oxidized sodium alginate at a concentration of 3% was formulated by dissolving the oxidized sodium alginate in the distilled water, a concentration of the aqueous solution of oxidized sodium alginate was made to 30 mg/ml by adding the glucose oxidase to the aqueous solution of oxidized sodium alginate, the concentration of the aqueous solution of oxidized sodium alginate was made to 20 mg/ml by adding bovine serum albumin to the aqueous solution of oxidized sodium alginate, and a pre-cross-linked glucose oxidase mixed solution was obtained by stirring and dissolving.

In the fifth step, an enzyme membrane layer was obtained by coating the above pre-cross-linked glucose oxidase mixed solution on the metal layer, cross-linking at 4°C for 24 h, and washing away uncross-linked glucose oxidase using distilled water.

In the sixth step, the protective layer was obtained by placing the enzyme membrane layer in a polyurethane solution at a concentration of 5%, taking out the enzyme membrane layer, and drying the enzyme membrane layer at room temperature, thereby obtaining the working electrode.

### Embodiment 2

In the first step, an aqueous solution of carboxymethyl cellulose was obtained by dissolving 5 g of carboxymethyl cellulose in 500 ml of distilled water.

In the second step, an aqueous solution of sodium periodate was obtained by dissolving 4.5 g of sodium periodate in 50 ml of distilled water.

In the third step, the aqueous solution of sodium periodate was added dropwise to the aqueous solution of carboxymethyl cellulose, the reaction was carried out in the dark at 4°C temperature for 24h, then the reaction was terminated by adding 5 ml of ethylene glycol and stirring for 1h, and then the oxidized carboxymethyl cellulose was obtained by filtering the aqueous solution and then drying in an oven.

In the fourth step, an aqueous solution of oxidized carboxymethyl cellulose at a concentration of 5% was formulated by dissolving the oxidized carboxymethyl cellulose in the distilled water, a concentration of the aqueous solution of oxidized carboxymethyl cellulose was made to 20 mg/ml by adding the glucose oxidase to the aqueous solution of oxidized carboxymethyl, the concentration of the aqueous solution of oxidized carboxymethyl alginate was made to 15 mg/ml by adding the bovine serum albumin to the aqueous solution of oxidized carboxymethyl, and a pre-cross-linked glucose oxidase mixed solution was obtained by stirring and dissolving.

In the fifth step, the enzyme membrane layer was obtained by coating the above pre-cross-linked glucose oxidase mixed solution on the metal layer, cross-linking at 4°C for 12 h, and washing away uncross-linked glucose oxidase using the distilled water.

In the sixth step, the protective layer was obtained by placing the enzyme membrane layer in a polyurethane solution at a concentration of 5%, taking out the enzyme membrane layer, and drying the enzyme membrane layer at room temperature, thereby obtaining the working electrode.

### Embodiment 3

In the first step, an aqueous solution of pectin was obtained by dissolving 1 g of pectin in 100 ml of distilled water.

In the second step, an aqueous solution of sodium periodate was obtained by dissolving 1 g of sodium periodatein 50 ml of distilled water.

In the third step, the aqueous solution of sodium periodate was added dropwise to the aqueous solution of pectin, the reaction was carried out in the dark at 4°C for 12h, then the reaction was terminated by adding 5 ml of ethylene glycol and stirring for 1h, and then the oxidized pectin was obtained by filtering the aqueous solution and then drying in an oven.

In the fourth step, an aqueous solution of oxidized pectin at a concentration of 1% was formulated by dissolving the oxidized pectin in the distilled water, a concentration of the aqueous solution of oxidized pectin was made to 10 mg/ml by adding the glucose oxidase to the aqueous solution of oxidized pectin, the concentration of the aqueous solution of oxidized pectin was made to 8 mg/ml by adding the bovine serum albumin to the aqueous solution of oxidized pectin, and the pre-cross-linked glucose oxidase mixed solution was obtained by stirring and dissolving.

In the fifth step, the enzyme membrane layer was obtained by coating the above pre-cross-linked glucose oxidase mixed solution on the metal layer, cross-linking at a 4°C for 24 h, and washing away the uncross-linked glucose oxidase using the distilled water.

In the sixth step, the protective layer was obtained by placing the enzyme membrane layer in a polyurethane solution at a concentration of 5%, taking out the enzyme membrane layer, and drying the enzyme membrane layer at room temperature, thereby obtaining the working electrode.

### Comparative Embodiment 1

The working electrodes obtained from Embodiments 1 to 3 and from the preparation in Comparative Embodiment 1 were respectively used to prepare the glucose biosensor. The method for preparing the glucose biosensor in the Comparative Embodiment 1 includes:

In the first step, polyimide (PI) with a thickness of 120 µm was selected as a flexible base, and glass or silicon support base and PI were cleaned and blown dry with a nitrogen gas gun.

In the second step, polydimethylsiloxane (PDMS) was spin-coated onto the support base and then heated to cure.

In the third step, the cleaned PI was adsorbed using a laminator and the support base coated with the PDMS.

In the fourth step, on the PI flexible base, a bonding layer of chromium (Cr) was sputtered using a magnetron sputtering apparatus, a conductive layer of gold (Au) was then sputtered, a layer of photoresist was then spin-coated, a patterning process was performed using an exposure machine, and then a development reaction was performed. The conductive layer of Au was wet etched, cleaned with a large amount of water, and then blown dry with nitrogen gas. The bonding layer of Cr was then etched until a non-patterned region was completely exposed with a PI color (reddish brown), cleaned with a large amount of water, and blown dry with nitrogen gas. Finally, the photoresist was peeled off and whether the wires were connected were checked.

In the fifth step, on the PI flexible base, poly-para-xylene deposition was performed, the photoresist was then spin-coated, a development for patterning was performed, and then a plasma treatment was performed such that a developed portion of the poly-para-xylene deposition was cleaned, and finally, the photoresist was peeled off and whether the wires are insulated was checked.

In the sixth step, the working electrode and counter electrode were prepared: firstly, a layer of photoresist was spin-coated on the PI flexible bases of the working electrode and the counter electrode, respectively, the patterning process was performed using an exposure machine, then the development reaction was performed, and then blown dry with nitrogen gas after being washed by water. On the PI flexible base, a bonding layer of Cr was sputtered using the magnetron sputtering apparatus, and then a conductive layer of platinum (Pt) was sputtered. The PI flexible bases were further soaked in in acetone and underwent ultrasound until patterns of the working electrode (WE) and the counter electrode (CE) were completely lifted away, then the PI flexible bases were washed using acetone, alcohol, water, and blown dry using the nitrogen gun. Further, conductivities of the WE, CE, and corresponding component holes (PAD) and insulation of the different wires were tested using a multimeter. Therefore, the working electrode and the counter electrode were obtained.

In the seventh step, the working electrode containing the enzyme membrane layer was prepared: a pre-cross-linked glucose oxidase mixed solution was obtained by dissolving the glucose oxidase and the bovine serum protein in a phosphate buffer solution (PBS) and adding 2.5% of glutaraldehyde solution to the PBS. The enzyme membrane layer was obtained by coating and covering the above pre-cross-linked glucose oxidase mixed solution on the metal layer of the working electrode, cross-linking at 4°C for 24 h, and washing the uncross-linked glucose oxidase using distilled water. A protective layer was formed by placing the working electrode including the enzyme membrane layer in a polyurethane solution at a concentration of 5%, taking out and drying the working electrode at room temperature, thereby obtaining a dry working electrode containing the enzyme membrane layer.

In the eighth step, the reference electrode was prepared: electrodeposition of a silver layer: the electrodeposition was performed in a constant current manner using a pulse current source device; chlorination processing: first treated with argon plasma for 5 minutes, then treated with a ferric chloride solution, and finally rinsed with water and blown dry with nitrogen to obtain the reference electrode.

The working electrodes obtained from Embodiments 1 to 3 were respectively used to prepare the glucose biosensor according to the preparation method of Comparative Embodiment 1.

### Embodiment 4: Sensitivity and stability testing of biosensors

(i) Sensitivity testing of the glucose biosensor using the working electrodes in Embodiments 1-3 was performed.

Specifically, five groups of glucose biosensors were obtained using the working electrodes in Embodiment 1 according to the method of Comparative Embodiment 1, and sensitivities of the five groups of biosensors were tested at glucose concentrations within a range of 0 to 30 mM.

Test results of the sensitivities of the biosensors prepared using the working electrodes in Embodiment 1 are shown in FIG. 3A. The test results illustrate that the five groups of glucose biosensors prepared using the working electrodes in Embodiment 1 realize linearity at a glucose concentration within a range of 0 to 30 mM, and variability among the five groups of glucose biosensors is below 5%.

The sensitivities of glucose biosensors prepared using the working electrodes in Embodiments 2-3 were tested. Specifically, glucose biosensors were prepared using the working electrodes in Embodiments 2-3 according to the method of Comparative Embodiment 1, and the sensitivities of the glucose biosensors were tested at a glucose concentration within a range of 0 to 20 mM.

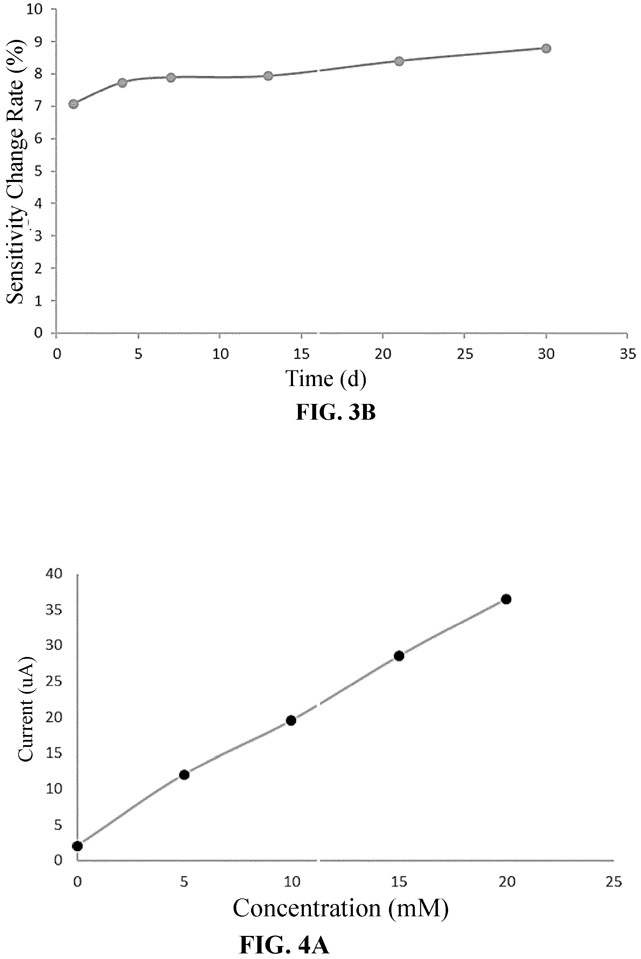

The test results of the sensitivities of the biosensors using the working electrodes in Embodiment 2 are shown in FIG. 4A, and the test result illustrates that the glucose biosensors using the working electrodes in Embodiment 2 realize the linearity at a glucose concentration within a range of 0 to 20 mM.

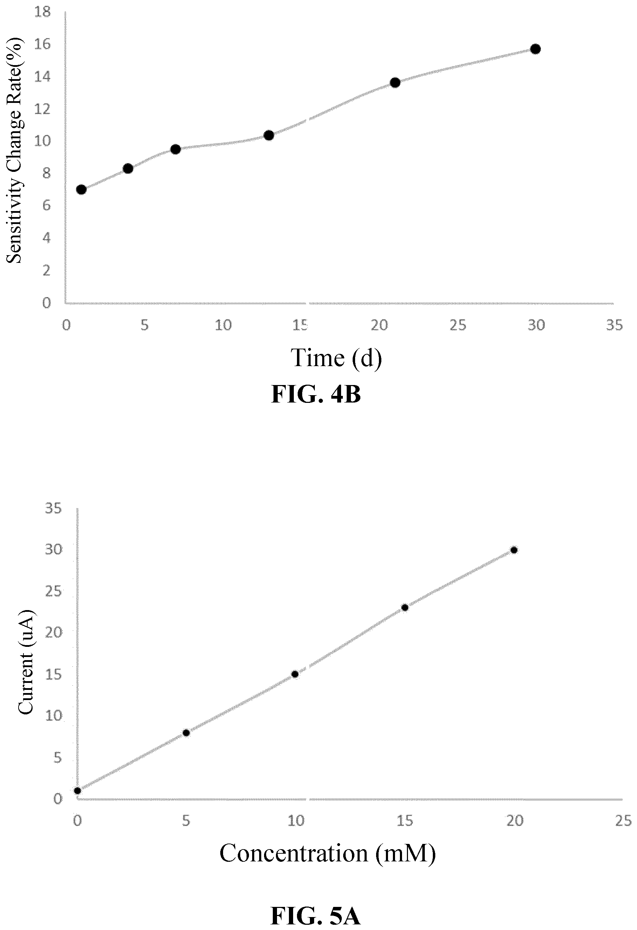

The test results of sensitivities of the biosensors using the working electrodes in Embodiment 3 are shown in FIG. 5A, and the test results illustrate that the glucose biosensors using the working electrodes in Embodiment 3 realize the linearity at a glucose concentration within a range of 0 to 20 mM.

(ii) Long-term stability testing of the glucose biosensors obtained using the working electrodes in Embodiments 1-3 was performed.

The test results of sensitivities of the biosensors using the working electrodes in Embodiment 1 are shown in FIG. 3B. The test results illustrate that a sensitivity variation of the glucose biosensors using the working electrodes in Embodiment 1 is less than 2% over 30 days. This indicates that the glucose biosensors prepared in the present disclosure have long-term stability.

The test results of sensitivities of the biosensors prepared using the working electrodes in Embodiment 2 are shown in FIG. 4B. The test results illustrate that the glucose biosensors prepared using the working electrodes in Embodiment 2 have a sensitivity variation of less than 10% over 30 days. This indicates that the glucose biosensors prepared in the present disclosure have long-term stability.

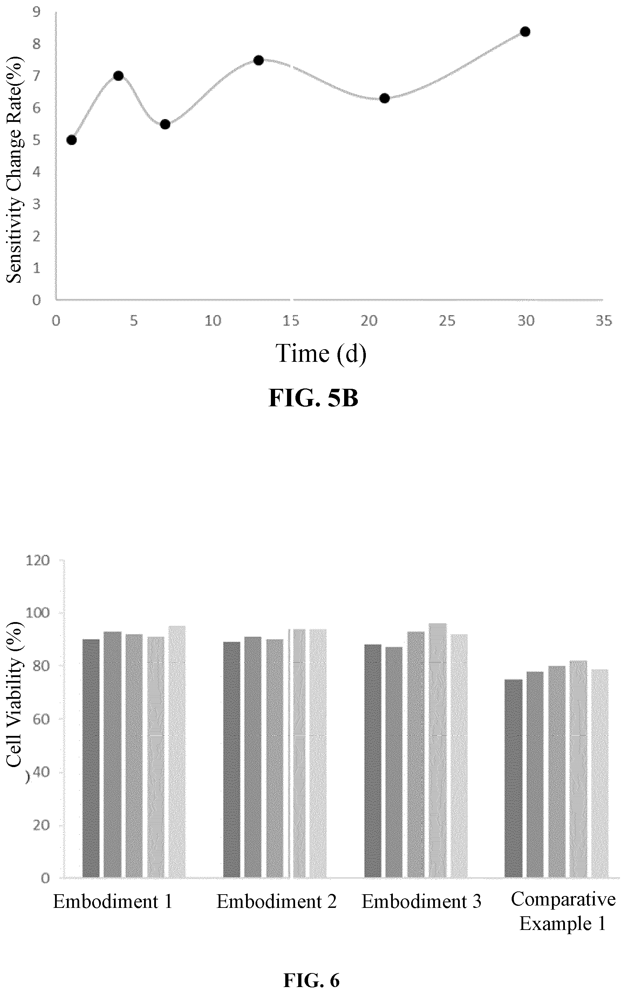

The test results of sensitivities of the biosensors using the working electrodes in Embodiment 3 are shown in FIG. 5B. The test results illustrate that the glucose biosensors using the working electrodes in Embodiment 3 have a sensitivity variation of less than 5% over 30 days. This indicates that the glucose biosensors prepared in the present disclosure have long-term stability.

According to the above test results, the biosensor obtained using the oxidized sodium alginate as the cross-linking agent of the enzyme membrane layer has the best stability. This may be because the molecular structure of the sodium alginate makes the cross-linking effect optimal, which ensures the count of sensitive moiety and that the sensitive moiety in the enzyme membrane layer is immobilized in the working electrode more stably.

### Embodiment 5: Cytotoxicity testing of working electrodes

Cytotoxicity tests were respectively performed on the working electrodes in Embodiments 1 to 3 and in Comparative Embodiment 1.

The cytotoxicity test of the working electrode includes: the working electrodes prepared in Embodiments 1 to 3 and Comparative Embodiment 1 were respectively soaked in PBS for leaching to obtain a leaching solution.

FIG. 6 is a diagram illustrating cytotoxicity test results of working electrodes according to some embodiments of the present disclosure.

Referring to FIG. 6, the test results illustrate that the survival rates of the cells are higher in the leaching solutions of the working electrodes prepared in Embodiments 1 to 3, while the survival rate of the cells is lower in the leaching solution of the working electrode prepared in Comparative Embodiment 1. This indicates that the toxicity of the oxidized natural polymer compound is lower than the toxicity of glutaraldehyde, thereby indicating that the use of the oxidized natural polymer compound as the cross-linking agent in the present disclosure is significantly superior to the use of glutaraldehyde as the cross-linking agent in the prior art.

The Beneficial effects of the embodiments of the present disclosure include but are not limited to:(1) By obtaining the oxidized natural polymer compound through oxidizing the natural polymer compound and using the oxidized natural polymer compound as the cross-linking agent for immobilizing the sensitive moiety in the working electrode of the biosensor, the biotoxicity of the conventional chemical cross-linking agent is prevented from generating, and the biocompatibility of the working electrode of the biosensor is improved. (2) By adjusting the process parameters in the oxidation reaction involving the natural polymer compound and the enzyme membrane layer preparation process, the environment and process controllability of the chemical bonding of the sensitive moieties are optimized and improved, which is conducive to maintaining the count and activity of the sensitive moieties, ensuring the activity and stability of the sensitive moieties, and thereby improving the consistency and the long-term stability of the biosensor.

It is to be noted that different embodiments may produce different beneficial effects, and in different embodiments, the beneficial effects that may be produced may be any one or a combination of any one or more of the above, or any other beneficial effect that may be obtained.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations thereof, are not intended to limit the claimed processes and methods to any order except as specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software-only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In closing, it is to be understood that the embodiments of the application disclosed herein are illustrative of the principles of the embodiments of the application. Other modifications that may be employed may be within the scope of the application. Therefore, by way of example, but not of limitation, alternative configurations of the embodiments of the application may be utilized in accordance with the teachings herein. Accordingly, embodiments of the present application are not limited to that precisely as shown and described.

Combinations of features which do not fall under the scope of the amended independent claims do not form part of the invention.

## Claims

1. A biosensor, including a working electrode, the working electrode including an enzyme membrane layer, wherein
the enzyme membrane layer includes a sensitive moiety immobilized with oxidized sodium alginate as a cross-linking agent, the oxidized sodium alginate is aldehydated, the sensitive moiety refers to an enzyme, and a thickness of the enzyme membrane layer is within a range of 1 µm to 5 µm.

2. The biosensor of claim 1, wherein the oxidized sodium alginate is a dialdehyde sodium alginate.

3. The biosensor of claim 2, wherein an oxidation degree of the oxidized sodium alginate is within a range of 5% to 85%, preferably within a range of 30% to 60%.

4. The biosensor of claim 1, wherein the sensitive moiety includes one of glucose oxidase, lactate oxidase, uricase, or alcohol oxidase.

5. The biosensor of claim 1, wherein the enzyme membrane layer further includes an enzyme stabilizer.

6. The biosensor of claim 1, further including at least one of a flexible base, a reference electrode, and a counter electrode.

7. The biosensor of claim 6, wherein a thickness of the flexible base is within a range of 10 µm to 300 µm.

8. The biosensor of claim 1, wherein the working electrode further includes at least one of a metal layer and a protective layer.

9. The biosensor of claim 8, wherein the metal layer of the working electrode includes a first metal layer, a second metal layer, and a third metal layer in order from inside to outside; wherein the first metal layer is at least one of Cr, Ti, and Ni-Cr; the second metal layer is at least one of gold, copper, and silver; and the third metal layer is at least one of platinum, platinum-iridium, and platinum-carbon.

10. The biosensor of claim 8, wherein the protective layer includes at least one of polyurethane, polyethylene glycol, cellulose acetate, polyvinyl alcohol, polyvinylpyrrolidone, and polyethylene.

11. A preparation method for the biosensor of any one of claims 1-10, including:
immobilizing the sensitive moiety with oxidized sodium alginate in the working electrode of the biosensor.

12. The preparation method of claim 11, wherein the method further includes obtaining the oxidized sodium alginate by performing an oxidation operation on a sodium alginate including:
obtaining a sodium alginate solution by dissolving the sodium alginate in a first solvent;
obtaining an oxidizing solution by dissolving an oxidizing agent in a second solvent; and
obtaining the oxidized sodium alginate based on an oxidation reaction performed by mixing the sodium alginate solution with the oxidizing solution.

13. The preparation method of claim 12, wherein the method further includes preparing the enzyme membrane layer by:
obtaining a mixed solution by mixing an enzyme stabilizer, the sensitive moiety, and the oxidized sodium alginate in a third solvent; and
obtaining the enzyme membrane layer based on a cross-linking reaction performed by coating the mixed solution on a metal layer.

14. The preparation method of claim 13, wherein in the mixed solution, a concentration of the sensitive moiety is within a range of 2% to 10%; a concentration of the oxidized sodium alginate is within a range of 5% to 15%; a concentration of the enzyme stabilizer is within a range of 0.5% to 5%; and a temperature of the cross-linking reaction is within a range of 5°C to 15°C.

## Patentansprüche

1. Biosensor, der eine Arbeitselektrode beinhaltet, wobei die Arbeitselektrode eine Enzymmembranschicht beinhaltet, wobei
die Enzymmembranschicht eine empfindliche Einheit beinhaltet, die mit oxidiertem Natriumalginat als Vernetzungsmittel immobilisiert ist, das oxidierte Natriumalginat aldehydiert ist, wobei sich die empfindliche Einheit auf ein Enzym bezieht und die Dicke der Enzymmembranschicht in einem Bereich von 1 µm bis 5 µm liegt.

2. Biosensor nach Anspruch 1, wobei es sich bei dem oxidierten Natriumalginat um ein Natriumalginat-Dialdehyd handelt.

3. Biosensor nach Anspruch 2, wobei der Oxidationsgrad des oxidierten Natriumalginats in einem Bereich von 5% bis 85%, vorzugsweise in einem Bereich von 30 % bis 60% liegt.

4. Biosensor nach Anspruch 1, wobei die empfindliche Einheit Glucoseoxidase, Lactatoxidase, Uricase oder Alkoholoxidase beinhaltet.

5. Biosensor nach Anspruch 1, wobei die Enzymmembranschicht weiter einen Enzymstabilisator beinhaltet.

6. Biosensor nach Anspruch 1, der weiter mindestens eine flexible Basis, eine Referenzelektrode und eine Gegenelektrode beinhaltet.

7. Biosensor nach Anspruch 6, wobei die Dicke der flexiblen Basis in einem Bereich von 10 µm bis 300 µm liegt.

8. Biosensor nach Anspruch 1, wobei die Arbeitselektrode weiter mindestens eine Metallschicht und eine Schutzschicht beinhaltet.

9. Biosensor nach Anspruch 8, wobei die Metallschicht der Arbeitselektrode von innen nach außen eine erste Metallschicht, eine zweite Metallschicht und eine dritte Metallschicht beinhaltet; wobei die erste Metallschicht aus mindestens einem von Cr, Ti und Ni-Cr besteht; die zweite Metallschicht aus mindestens einem von Gold, Kupfer und Silber besteht; und die dritte Metallschicht aus mindestens einem von Platin, Platin-Iridium und Platin-Kohlenstoff besteht.

10. Biosensor nach Anspruch 8, wobei die Schutzschicht mindestens eines von Polyurethan, Polyethylenglykol, Celluloseacetat, Polyvinylalkohol, Polyvinylpyrrolidon und Polyethylen beinhaltet.

11. Herstellungsverfahren für den Biosensor nach einem der Ansprüche 1-10, das beinhaltet:
Immobilisieren der empfindlichen Einheit mit oxidiertem Natriumalginat in der Arbeitselektrode des Biosensors.

12. Herstellungsverfahren nach Anspruch 11, wobei das Verfahren weiter Erhalten des oxidierten Natriumalginats durch Durchführen eines Oxidationsvorgangs an einem Natriumalginat beinhaltet, beinhaltend:
Erhalten einer Natriumalginatlösung durch Auflösen des Natriumalginats in einem ersten Lösungsmittel;
Erhalten einer oxidierenden Lösung durch Auflösen eines Oxidationsmittels in einem zweiten Lösungsmittel; und
Erhalten des oxidierten Natriumalginats auf Grundlage einer Oxidationsreaktion, die durch Mischen der Natriumalginatlösung mit der oxidierenden Lösung durchgeführt wird.

13. Herstellungsverfahren nach Anspruch 12, wobei das Verfahren weiter Herstellen der Enzymmembranschicht beinhaltet durch:
Erhalten einer gemischten Lösung durch Mischen eines Enzymstabilisators, der empfindlichen Einheit und des oxidierten Natriumalginats in einem dritten Lösungsmittel; und
Erhalten der Enzymmembranschicht auf Grundlage einer Vernetzungsreaktion, die durch Auftragen der gemischten Lösung auf eine Metallschicht durchgeführt wird.

14. Herstellungsverfahren nach Anspruch 13, wobei in der gemischten Lösung die Konzentration der empfindlichen Einheit in einem Bereich von 2% bis 10% liegt; die Konzentration des oxidierten Natriumalginats in einem Bereich von 5% bis 15% liegt; die Konzentration des Enzymstabilisators in einem Bereich von 0,5 % bis 5% liegt und die Temperatur der Vernetzungsreaktion in einem Bereich von 5°C bis 15°C liegt.

## Revendications

1. Biocapteur, incluant une électrode de travail, l'électrode de travail incluant une couche de membrane enzymatique, dans lequel
la couche de membrane enzymatique inclut une fraction sensible immobilisée avec de l'alginate de sodium oxydé comme agent de réticulation, l'alginate de sodium oxydé est aldéhyde, la fraction sensible fait référence à une enzyme et une épaisseur de la couche de membrane enzymatique est dans une plage de 1 µm à 5 µm.

2. Biocapteur selon la revendication 1, dans lequel l'alginate de sodium oxydé est un alginate de sodium dialdéhyde.

3. Biocapteur selon la revendication 2, dans lequel le degré d'oxydation de l'alginate de sodium oxydé est dans une plage de 5 % à 85 %, de préférence dans une plage de 30 % à 60 %.

4. Biocapteur selon la revendication 1, dans lequel la fraction sensible inclut un parmi le glucose oxydase, le lactate oxydase, l'uricase ou l'alcool oxydase.

5. Biocapteur selon la revendication 1, dans lequel la couche de membrane enzymatique inclut en outre un stabilisateur d'enzyme.

6. Biocapteur selon la revendication 1, incluant en outre au moins une base flexible, une électrode de référence et une contre-électrode.

7. Biocapteur selon la revendication 6, dans lequel une épaisseur de la base flexible est dans une plage de 10 µm à 300 µm.

8. Biocapteur selon la revendication 1, dans lequel l'électrode de travail inclut en outre au moins une couche métallique et une couche protectrice.

9. Biocapteur selon la revendication 8, dans lequel la couche métallique de l'électrode de travail inclut une première couche métallique, une deuxième couche métallique et une troisième couche métallique, dans l'ordre de l'intérieur vers l'extérieur; dans lequel la première couche métallique est au moins une parmi Cr, Ti et Ni-Cr ; la deuxième couche métallique est au moins une parmi l'or, le cuivre et l'argent ; et la troisième couche métallique est au moins une parmi le platine, le platine-iridium et le platine-carbone.

10. Biocapteur selon la revendication 8, dans lequel la couche protectrice inclut au moins un parmi le polyuréthane, le polyéthylène glycol, l'acétate de cellulose, l'alcool polyvinylique, le polyvinylpyrrolidone et le polyéthylène.

11. Procédé de préparation pour le biocapteur selon l'une quelconque des revendications 1-10, incluant :
l'immobilisation de la fraction sensible avec de l'alginate de sodium oxydé dans l'électrode de travail du biocapteur.

12. Procédé de préparation selon la revendication 11, dans lequel le procédé inclut en outre l'obtention de l'alginate de sodium oxydé par la réalisation d'une opération d'oxydation sur un alginate de sodium incluant :
l'obtention d'une solution d'alginate de sodium par dissolution de l'alginate de sodium dans un premier solvant ;
l'obtention d'une solution oxydante en dissolvant un agent oxydant dans un deuxième solvant ; et
l'obtention de l'alginate de sodium oxydé sur la base d'une réaction d'oxydation réalisée en mélangeant la solution d'alginate de sodium avec la solution oxydante.

13. Procédé de préparation selon la revendication 12, dans lequel le procédé inclut en outre la préparation de la couche de membrane enzymatique par :
obtention d'une solution mixte en mélangeant un stabilisateur enzymatique, la fraction sensible et l'alginate de sodium oxydé dans un troisième solvant ; et
obtention de la couche de membrane enzymatique sur la base d'une réaction de réticulation réalisée par revêtement de la solution mixte sur une couche métallique.

14. Procédé de préparation selon la revendication 13, dans lequel, dans la solution mixte, une concentration de la fraction sensible est dans une plage de 2 % à 10 % ; une concentration de l'alginate de sodium oxydé est dans une plage de 5 % à 15 % ; une concentration du stabilisateur enzymatique est dans une plage de 0,5 % à 5 % ; et une température de la réaction de réticulation est dans une plage de 5 °C à 15 °C.
